# EUROPEAN PATENT SPECIFICATION

(11) **EP 3 827 874 B1**
(45) Date of publication and mention of the grant of the patent: **28.10.2026**
(21) Application number: 20215150.2
(22) Date of filing: 11.11.2014
(51) Int. Cl.: A61N 1/05, A61N 1/36

(54) **COMPUTER-IMPLEMENTED METHOD, PROCESSOR, AND SYSTEM FOR DETERMINING ELECTRODE NEUROMODULATION SETTINGS**
COMPUTERIMPLEMENTIERTES VERFAHREN, PROZESSOR UND SYSTEM ZUR BESTIMMUNG DER NEUROMODULATIONSEINSTELLUNGEN VON ELEKTRODEN
PROCÉDÉ MIS EN OEUVRE PAR ORDINATEUR, PROCESSEUR ET SYSTÈME POUR DÉTERMINER DES PARAMÈTRES DE NEUROMODULATION D'ÉLECTRODES

(30) Priority: 14.11.2013 US 201361904248 P; 01.10.2014 US 201462058131 P
(43) Date of publication of application: 02.06.2021
(62) Document number(s) of the earlier application(s) in accordance with Art. 76 EPC: 14809172.1 / 3 068 483
(73) Proprietor: Boston Scientific Neuromodulation Corporation, Valencia, CA 91355 (US)
(72) Inventor: MOFFITT, Michael A., Valencia, CA 91354 (US); CARBUNARU, Rafael, Valley Village, CA 91067 (US)
(74) Representative: Vossius & Partner Patentanwälte Rechtsanwälte mbB

(56) References cited:
- US-A1- 2007 067 003
- US-A1- 2007 083 104
- US-A1- 2012 014 580
- US-A1- 2012 265 261
- US-A1- 2013 030 276
- US-A1- 2013 150 922

## Description

### FIELD OF THE INVENTION

Aspects of the present invention are directed to a system(s) and method(s) for sensing and anatomical stimulation by electrodes of an implanted leadwire, where the stimulation is programmable to be responsive to a sensed signal. Aspects of the present invention are directed to a system for generating a patient-specific interaction model, determining a relationship between stimulation paradigms, including stimulation sites, and neuromodulation sites, dynamically changing stimulation goals and paradigms in response to sensed changes to a state, and/or outputting visual representations of a correlation between candidate stimulation sites and affected anatomical regions. Aspects of the present invention are directed to a system configured to perform stimulations and sense or receive input of data concerning effects of the stimulations to generate a patient-specific interaction model usable by the system to determine the sites to be stimulated for causing neuromodulation at sites selected by a user and for outputting a visual mapping of relationships between candidate stimulation sites and the affected neuromodulation sites. Aspects of the present invention are directed to generating a patient-specific interaction model based on tractography analysis prior to implantation of a leadwire, the model indicating various relationships between candidate stimulation sites and the affected neuromodulation sites for different placements of the leadwire, the model being output for use by a user to pre-operatively determine an optimal implantation site for the leadwire. The system is usable both for clinical use, and for analysis and scientific investigation. The present invention is further related to subject matter of U.S. Pat. App. Ser. No. 13/160,104, published as U.S. Pat. App. Pub. No 2012/0014580.

### BACKGROUND INFORMATION

Stimulation of anatomical regions of a patient is a clinical technique for the treatment of disorders. Such stimulation can include deep brain stimulation (DBS), spinal cord stimulation (SCS), Occipital NS therapy, Trigemenal NS therapy, peripheral field stimulation therapy, sacral root stimulation therapy, or other such therapies. For example, DBS may include electrical stimulation of the thalamus or basal ganglia and may be used to treat disorders such as movement disorders such as essential tremor, Parkinson's disease (PD), and dystonia, and other physiological disorders. DBS may also be useful for traumatic brain injury and stroke. DBS may also be useful for treating depression, obesity, epilepsy, and obsessive-compulsive disorder, Tourette's Syndrome, schizophrenia, and other indications.

A stimulation procedure, such as DBS, typically involves first obtaining preoperative images, e.g., of the patient's brain, such as by using a computed tomography (CT) scanner device, a magnetic resonance imaging (MRI) device, or any other imaging modality. This sometimes involves first affixing to the patient's skull spherical or other fiducial markers that are visible on the images produced by the imaging modality. The fiducial markers help register the preoperative images to the actual physical position of the patient in the operating room during the later surgical procedure.

After the preoperative images are acquired by the imaging modality, they are then loaded onto an image-guided surgical (IGS) workstation, and, using the preoperative images displayed on the IGS workstation, a neurosurgeon can select a target region within the patient anatomy, e.g., within the brain, an entry point, e.g., on the patient's skull, and a desired trajectory between the entry point and the target region. The entry point and trajectory are typically carefully selected to avoid intersecting or otherwise damaging certain nearby critical structures or vasculature.

In the operating room, the physician marks the entry point on the patient's skull, drills a burr hole at that location, and affixes a trajectory guide device about the burr hole. The trajectory guide device includes a bore that can be aimed to obtain the desired trajectory to the target region. After aiming, the trajectory guide is locked to preserve the aimed trajectory toward the target region, and a microdrive introducer is then used to insert the surgical instrument along the trajectory toward the target region, e.g., of the brain. The surgical instrument may include, among other things, a recording electrode leadwire, for recording intrinsic electrical signals, e.g., of the brain; a stimulation electrode leadwire, for providing electrical energy to the target region, e.g., of the brain; or associated auxiliary guidewires or guide catheters for steering a primary instrument toward the target region, e.g., of the brain.

The stimulation electrode leadwire, which typically includes multiple closely-spaced electrically independent stimulation electrode contacts, is then introduced and positioned in close proximity to the tissue targeted for sitmulation, to deliver the therapeutic stimulation to the target region, e.g., of the brain. An implanted pulse generator (IPG) generates electric pulses to transmit signals via the leadwire. The leadwire can include cylindrically symmetrical electrodes, which, when operational, produce approximately the same electric values in all positions at a same distance from the electrode in any plain that cuts through the electrode perpendicular to the central longitudinal axis of the leadwire. Alternatively, the leadwire can include directional electrodes that produce different electrical values depending on the direction from the electrode. The stimulation electrode leadwire is then immobilized, such as by using an instrument immobilization device located at the burr hole entry, e.g., in the patient's skull, in order for the DBS therapy to be subsequently performed.

The target anatomical region can include tissue that exhibit high electrical conductivity. For given stimulation parameter settings, a respective subset of the neural elements are responsively activated. A stimulation parameter can include, for example, a current amplitude or voltage amplitude, which may be the same for all of the electrodes of the leadwire, or which may vary between different electrodes of the leadwire. The applied amplitude setting results in a corresponding current in the surrounding neural elements, and therefore a corresponding voltage distribution in the surrounding tissue.

After the immobilization of the stimulation electrode leadwire, the actual stimulation therapy is often not initiated until after a time period of about two-weeks to one month has elapsed. This is due primarily to the acute reaction of the brain tissue to the introduced electrode leadwire (e.g., the formation of adjacent scar tissue), and stabilization of the patient's disease symptoms. At that time, a particular one or more of the stimulation electrode contacts is selected for delivering the therapeutic stimulation, and other stimulation parameters are adjusted to achieve an acceptable level of therapeutic benefit. The IPGs offer a wide range of stimulation settings which can be independently or concurrently varied in order to correspondingly alter the size, shape, and location of the volume of tissue being therapeutically affected by the stimulation.

Systems and methods are provided that facilitate exploration of target regions of stimulation and stimulation therapies to determine which therapy regimen is best suited for a particular patient or group of patients.

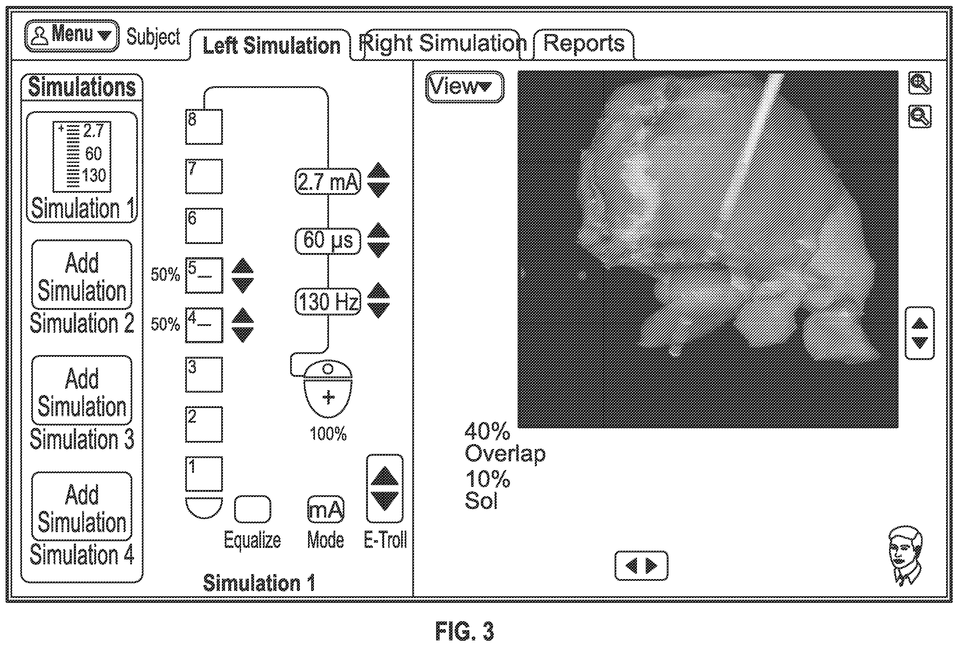

A treating physician typically would like to tailor the stimulation parameters (such as which one or more of the stimulating electrode contacts to use, the stimulation pulse amplitude, e.g., current or voltage depending on the stimulator being used, the stimulation pulse width, and/or the stimulation frequency) for a particular patient to improve the effectiveness of the therapy. Parameter selections for the stimulation can be achieved, for example, via trial-and-error. However, the use of guiding visualization software provides for efficient stimulation parameter selection. *See* Frankemolle, A. et al., "Reversing cognitivemotor impairments in Parkinson's disease patients using a computational modelling approach to deep brain stimulation programming," Brain 133 (3): 746-761 (2010). Indeed, systems and methods are provided that provide visual aids of the electrode location in the tissue medium along with computational models of the volume of tissue influenced by the stimulation, thereby facilitating parameter selection. See, for example, U.S. Pat. App. Ser. No. 12/454,330, filed May 15, 2009, which published as U.S. Pat. App. Pub. No. 2009/0287271 ("the '330 application"), U.S. Pat. App. Ser. No. 12/454,312, filed May 15, 2009, which issued as U.S. Pat. No. 8,326,433 ("the '312 application"), U.S. Pat. App. Ser. No. 12/454,340, filed May 15, 2009, which published as U.S. Pat. App. Pub. No. 2009/0287272 ("the '340 application"), U.S. Pat. App. Ser. No. 12/454,343, filed May 15, 2009, which published as U.S. Pat. App. Pub. No. 2009/0287273 ("the '343 application"), and U.S. Pat. App. Ser. No. 12/454,314, filed May 15, 2009, which published as 2009/0287467 ("the '314 application"). Those applications describe systems including equation-based models for generation of estimated volumes of activation (VOAs) based on input of stimulation parameters. The described systems and methods provide for estimation of stimulation volumes and display models of a patient anatomy and/or a stimulation leadwire, via which to graphically identify the estimated stimulation volumes and how they interact with various regions of the patient anatomy. If a physician selects a therapeutic stimulation parameter combination, the software displays a representation of the volume of surrounding tissue which is estimated to be activated by the system. *See also* S. Miocinovic et al., "Cicerone: stereotactic neurophysiological recording and deep brain stimulation electrode placement software system," Acta Neurochir. Suppl. 97(2): 561 -567 (2007). Fig. 3 shows an example user interface, using which a user can input and/or modify stimulator settings in the left two panels, while the right panel shows a model of anatomical structures, an implanted leadwire, and an estimated VOA.

U.S. Prov. Pat. App. Ser. Nos. 61/521,583 ("the '583 application"), filed August 9, 2011 and 61/690,270 ("the '270 application"), filed June 22, 2012, and U.S. Pat. App. Ser. No. 13/507,962, filed August 9, 2012, which published as U.S. Pat. App. Pub. No. 2013/0116744 ("the '962 application"), further describe generation of a VOA on a fiber specific basis.

US 2013/0030276 A1 discloses techniques in which volumetric imaging data representing an anatomical volume of a brain of a patient can be obtained and transformed to brain atlas data. A patient-specific brain atlas can be created using the inverse of the transformation to map the brain atlas data onto the volumetric imaging data and a volume of influence can be calculated using the patient-specific brain atlas.

US 2012/0014580 A1, US 2007/0083104 A1, US 2013/0150922 A1, and US 2012/0265261 A1 disclose further exemplary stimulation devices

### SUMMARY OF THE INVENTION AND OF THE PRESENT DISCLOSURE

The invention is defined by the appended independent claims 1, 6 and 7. Further embodiments of the claimed invention are described in the appended dependent claims. Any "aspect", "embodiment", or "example" described in the following and not falling within the scope of the claimed invention thus defined should therefore be regarded as background information that is provided to facilitate the understanding of the claimed invention.

Disclosed herein is a stimulation leadwire apparatus that includes a plurality of leadwires connected to a programming source, e.g., an implanted pulse generator (IPG) including a plurality of ports to the leadwires, where the leadwires include a multitude of electrodes. In an example, the arrangement further includes multiplexers by which to select particular ones of the leadwires and electrodes. The electrodes, or at least a portion thereof, are controllable for emitting electrical pulses to stimulate an anatomical region, e.g., the brain, of a patient in whom the leadwire is implanted. Additionally, the electrodes, or at least a portion thereof, according to one example, are usable as sensors for sensing effects of the stimulation. Because of the inclusion of a multitude of leadwires and electrodes, the electrodes can be placed at many different locations with stimulations and sensing performed at the many locations, by which much information can be accumulated, based on which the system, according to an example, generates a patient-specific interaction model that associates stimulation sites and their corresponding stimulation parameters to affected neuromodulation sites. For example, the system controls the electrodes to perform a stimulation, obtains sensor readings from the electrodes, and modifies the stimulation parameters according to the sensor readings. The system uses sensor information obtained over a plurality of such applied stimulations to generate and/or update the patient-specific interaction model. Further, in an example, the system modifies the stimulation parameters based on the patient-specific interaction model which has been updated according to the sensor information indicating effects of the prior stimulations. However, as explained below, the patient-specific interaction model can alternatively or as a supplement be generated based on other input such as a tractography map.

The present invention provides a computer-implemented method that simplifies parameter selection so that a system with a multitude of implanted electrodes positioned at a plurality of anatomical locations is not too unwieldy. For example, the system uses models by which a user can select a desired outcome at a neuromodulation effect region, and the system selects the particular electrodes to implement and the particular settings to use for those electrodes, so that the user need not select parameters for each of the multitude of electrodes. Thus, the mappings allow for implementation of such a system that includes a multitude of electrodes.

The present disclosure further provides a sensing and stimulation system that is configured to stimulate and sense at multiple sites of a neural system simultaneously. The system is configured for the stimulation to be performed either in an open-loop mode or a closed-loop mode in which the stimulation is in response to raw or processed sensed information. The system includes one or more modules for stimulating one or more anatomical regions, tissue, and/or structure. The one or more modules are configured to simultaneously sense in multiple sensing sites and stimulate one or more stimulation sites.

According to an example, the system is configured to obtain sensor information and/or user input for determining a state of the patient and/or a state of the system, and, based on such information in association with stimulation information, generate a transfer function, also referred to herein as a patient-specific interaction model, that associates stimulation sites and the associated sitmulation parameters with affected neuromodulation sites.

For example, the system stimulates a plurality of anatomical sites, one by one, at one stimulation parameter set or at multiple stimulation parameter sets. For each such stimulation, the system uses information obtained from sensors and/or from user input information regarding the effect of the respective stimulations on the patient, thereby learning a correlation of stimulation sites to affected neuromodulation sites, such correlation forming a patient-specific interaction model that predicts affected neuromodulation sites for particular stimulation sites (e.g., which can differ for different stimulation parameters). The system is configured to use the interaction model to thereafter output a suggested stimulation site (and stimulation parameters) in response to user input of one or more targeted neuromodulation regions. For example, based on the known transfer function, the system uses an optimization algorithm to determine the suggested one or more stimulation sites and one or more parameter sets for effecting the neuromodulation at the one or more target sites, which may be different than the stimulation sites. For example, the system initially assumes a linear expandability of the interaction model to obtain initial candidates and then refines the candidate selection using any suitably appropriate optimization, e.g., a gradient descent optimization algorithm.

According to an example, the system is configured to stimulate one or more sites and sense (e.g., local field potentials (LFP), action potentials at individual neurons, mean firing rate, power at a particular frequency bands) either simultaneously or sequentially at multiple sites, and, according to an example, the system is configured to modify the stimulation at the one or more sites based on the sensing at the multiple sites. Epilepsy is one of the only indications where stimulation responsive to sensing has been shown to have value. In spinal cord stimulation (SCS), sensing has also been shown to have value (Saluda/NICTA) for managing the amplitude for a patient. Clinical evaluations are being performed, and the system can help perform evaluations, to determine further value in such sensing. Specifically, examples provide for sensing at the multiple sites and/or using sensed action potentials, mean firing rate, and/or frequency band specific power, as mentioned, which provide an enhanced closed-loop operation.

In an example, the system further includes a module for visualizing (and/or outputting a listing of) areas and/or components of the neural system expected to be affected by stimulation at the one or more stimulation sites. The module is configured to determine the expected sites based on a system model (the model can be derived from patient-specific radiographic data, an atlas-like model, information derived from evoked responses to stimulation as measured by the system, information compiled from similar data into an average-patient model, or information derived from PET, MRI, or other functional imaging data).

The system, according to examples, includes algorithms for automated determination of interaction models, and to automatically derive stimulation solutions to a user defined stimulation goal, e.g., stimulation of areas X and Y and inhibition of A and B to achieve a particular goal) (open-loop or closed-loop in response to an observed signature (e.g., LFP, mean firing rate, etc.)). Further in this regard, the use-input goal can be to produce certain neuromodulation effects and/or to reduce neural activity in one or more regions. For example, a sensor might sense neural activity in one or more regions which neural activity a clinician can input should be reduced. The system can then use an optimization algorithm to output at which one or more regions to perform a stimulation, and with which stimulation parameters, to most effectively achieve the indicated neuromodulation at the indicated sites and to most effectively reduce the indicated neural effects (e.g., at the indicated regions).

Further, the system is highly scalable, and therefore can be used to interact with complex multi-site neural systems (of which type most neural systems are), and for new and improved therapies in response to advancements in spatio-temporal interaction with the nervous system.

According to examples of the present disclosure, a leadwire includes more than 16 electrodes and the system is configured to control stimulation using such a leadwire. According to an example, the system is scalable to provide, control, and monitor hundreds of electrodes.

According to an example, the system provides a neural system visualization aid, which can help a clinician adjust the complex stimulation program.

According to an aspect of the claimed invention as defined by the appended independent claim 1, a computer-implemented method includes generating or obtaining a mapping between neuromodulation effect regions of a patient and target anatomical stimulation candidate regions; receiving, by a computer processor, user input selecting one of the neuromodulation effect regions of the patient; using, by the computer processor, the mapping to map at least one target anatomical stimulation candidate region to the selected one of the neuromodulation effect regions; and responsive to the input, determining, by the computer processor, electrode neuromodulation settings estimated by the processor to, when applied to an implanted electrode leadwire, produce a volume of tissue activation that at least partially encompasses at least one target anatomical stimulation candidate region which is mapped by the computer processor to, and is at a distance from, the selected neuromodulation effect region. According to the claimed invention, the mapping: is based on a probabilistic tractography model which is based on a susceptibility weighted imaging, SWI, image, wherein the computer processor performs an estimation of stimulation effect based on the probabilistic tractography model; and/or includes a patient-specific mapping that is specific to a patient and that is based on response information indicating prior responses by the patient to previous neuromodulations, wherein the response information includes sensor information sensed by at least one sensor configured for sensing patient responses to stimulation.

According to an example aspect of the method, the leadwire is implanted in the patient's brain. Alternatively or additionally, the at least one target anatomical stimulation candidate region includes a plurality of distinct regions. Alternatively or additionally, the user input specifies the neuromodulation effect region as the region at which neural activity is to be reduced.

According to an example aspect of the method, which can be provided in combination with any one or more of the above-noted aspects, the selected neuromodulation effect region is one of a plurality of neuromodulation effect regions selected by the user input, and the determining includes determining one or more sets of neuromodulation settings to be distributed to one or more electrodes of the leadwire to produce a selected therapeutic effect at the plurality of neuromodulation effect regions.

According to an example aspect of the method, which can be provided in combination with any one or more of the above-noted aspects, the mapping performed by the processor is performed by extrapolating new stimulation parameters from modeled stimulation parameters that are mapped in the model to neuromodulation effect regions, which stimulation parameters are estimated, by the processor and based on the model, to produce a selected effect at the selected neuromodulation effect region, the selected effect at the selected neuromodulation effect region not being mapped in the model.

Further disclosed herein is a stimulation system includes an implanted pulse generator (IPG) including a plurality of ports; a plurality of leadwires, each of the leadwires including at least one respective electrode; and at least one multiplexer between a respective one of the ports and a respective subset of more than one of the plurality of leadwires, which multiplexer is configured to select between the subset of the more than one of the plurality of leadwires for application to the electrodes thereof, of a signal from the respective port, the signal causing the electrodes thereof to produce electrical pulses for neuromodulation of tissue in which the subset of leadwires is implanted.

The various components and methods described herein may be practiced and/or provided, each alone, or in various combinations.

An further aspect of the present invention as defined by independent claim 6 is directed to a processor configured to perform an embodiment of the claimed computer-implemented method. The processor can be implemented using any conventional processing circuit and device or combination thereof, e.g., a Central Processing Unit (CPU) of a Personal Computer (PC) or other workstation processor, to execute code provided, e.g., on a hardware computer-readable medium including any conventional memory device, to perform any of the embodiments of the claimed method described herein. The memory device can include any conventional permanent and/or temporary memory circuits or combination thereof, a non-exhaustive list of which includes Random Access Memory (RAM), Read Only Memory (ROM), Compact Disks (CD), Digital Versatile Disk (DVD), and magnetic tape.

Further disclosed is a hardware computer-readable medium, e.g., as described above, having stored thereon instructions executable by a processor to perform the methods described herein.

Also disclosed is a method, e.g., of a hardware component or machine, of transmitting instructions executable by a processor to perform the methods described herein.

### BRIEF DESCRIPTION OF THE DRAWINGS

Fig. 1 illustrates a sensing and stimulation device, according to an example embodiment of the present invention.
Fig. 2 illustrates a sensing and stimulation device with multiplexing modules, according to an example embodiment of the present invention.
Fig. 3 is a conventional user interface for setting and visualizing stimulation programs.
Fig. 4 shows an example display of a user interface showing a tractographical map and stimulation model according to an example embodiment of the present invention, where, although not shown in the grayscale image, different displayed neural element are displayed in different colors, where the different colors represent a corresponding neuromodulation effect.
Fig. 5 shows a probabilistic patient-specific tractography-based model showing an expected neuromodulation effect at sites A-C (columns in the illustrated chart) for stimulations at respective ones of the sites A-C (rows in the illustrated chart), according to an example embodiment of the present invention.
Fig. 6 shows a probabilistic patient-specific biopotential-based model showing an expected neuromodulation effect at sites A-C (columns in the illustrated chart) for stimulations at respective ones of the sites A-C (rows in the illustrated chart), according to an example embodiment of the present invention.
Fig. 7 shows an example user interface display that graphically identifies areas likely to be affected by a particular stimulation paradigm, according to an example embodiment of the present invention.
Fig. 8 shows an example data flow for the system to propose a stimulation paradigm and associated parameters in response to user input, according to an example embodiment of the present invention.

### DETAILED DESCRIPTION

According to an example embodiment, a system includes at least one active stimulation/sensing module with a power source.

According to an example embodiment, the system includes more than one module, providing scalability. A plurality of the modules can be powered by a primary module or each can have its own power source. In an example embodiment, the multiple modules are interconnected by wires (or another tangible connection). Alternatively, the modules are wirelessly connected. Still further, according to an example embodiment, the system includes both wired and wireless connections between the modules.

According to an example embodiment, the system includes and manages stimulation and sensing of one or more leadwires that include more than 16 sensing/recording electrodes. A multiple current sources architecture (as opposed to a single voltage or current source for all of the electrodes) is the optimal stimulation architecture because it provides the best opportunity for stable stimulation, and enables fine tuning of stimulation at one or more stimulation sites.

According to example embodiments of the present invention, the system is configured to record at multiple locations simultaneously (or nearly simultaneously via high sampling rates) and stimulate at least one site. Sensed data from the multiple sites can be processed together to drive stimulation at the one or more sites.

Thus, for example, according to an example of the present invention, a method includes controlling electrodes of an implanted leadwire to perform a neuromodulation therapy according to a set of stimulation parameters; obtaining sensor readings from the electrodes; and responsive to the sensor readings, modifying the stimulation parameters. Further, according to an example, the modification is based on sensor readings of a plurality of the electrodes positioned at a respective plurality of anatomical locations. Below, a leadwire system particularly suitable for use to perform the sensing and stimulating is described, where the system includes electrodes positioned at many anatomical locations for sensing and/or stimulating.

The system also supports stimulation programmed in an open-loop mode. The system also supports sensing in an open-loop mode, where the system provides the user with sensed data (raw or processed) via a user interface, which data can help the user know if an electrode is in the right location (e.g., via knowledge of evoked potential signatures), and otherwise help the clinician/physician interact with the neural system. Note that the stimulation response can be programmed to occur in one or multiple places that can, but need not, coincide with the sensing site(s).

The utility of a system with such complexity is greatly enhanced by visualization and algorithms. Thus, according to example embodiments of the present invention, the system provides visualization components which help the physician to estimate how stimulation at one site affects other sites. According to an example embodiment, the system performs an estimation of stimulation effect (which it visualizes) based on patient-specific data, such as a probabilistic tractography model based on a diffusion tensor imaging (DTI) or susceptibility weighted imaging (SWI) image. According to an example embodiment, the tractography model is combined with a computational model of stimulation so that the effect of stimulation parameters (e.g., pulse width (PW), amplitude, rate, field configuration in terms of, e.g., anode/cathode and percentages) is included in the visualized estimate. In other words, a computation stimulation model is used to determine an electric field, activation thresholds, and therefore an immediate estimated activation region, and then the tractography model is applied to the computation stimulation model to determine which neural elements are in the immediate estimated activation region, the system following the extension of such neural elements to determine outlying sites which would be affected by those neural elements. In an example embodiment, the computational model includes linear (e.g., a simple model that fits definition of linear systems) or non-linear (e.g., including a differential equation and a solution thereof) models of representative neural elements.

According to an example embodiment, in instances where a patient-specific tractography data set is not available, the system is configured to register another tractography model or atlas (e.g., a patient population model or atlas that is registered to the patient) to create the estimate for a given patient. Other radiographic elements can be used to contribute to the interaction model (e.g., functional imaging such as fMRI, PET, EEG, MEG, etc.). For example, according to an example embodiment, the system generates a tractography model based on MR imaging.

In an example embodiment, the system allows the user to select points in the brain where neuromodulation is desired, and executes an algorithm that identifies candidate stimulation sites that are likely to affect one or more of the brain areas that the user desires to neuromodulate. In an example embodiment, the system produces an output for the candidate sites to be visualized by the user, and uses different colors (or other visual properties) to identify locations connected to a given brain area that the user has identified as a neuromodulation target. One advantage of the tool is that locations can be identified that are likely to enable neuromodulation of multiple candidate targets, i.e., "nodes" that represent candidate surgical and stimulation targets for achieving the input goals, which can include neuromodulation targets and/or sites at which certain neural activity is to be inhibited. These tools are likely to be helpful pre-operatively, when the clinician is working to identify stimulation targets for a specific patient.

Thus, according to an example, a method includes receiving, by a computer processor, user input selecting a neuromodulation effect region of a patient; and responsive to the input, determining, by the processor, electrode neuromodulation settings estimated by the processor to, when applied to an implanted electrode leadwire, produce a volume of tissue activation that at least partially encompasses at least one target anatomical stimulation candidate region which is mapped by the processor to, and is at a distance from, the selected neuromodulation effect region. In an example, the user-selected points can be at a plurality of distinct regions, which the processor determines would be affected by one or more direct stimulation sites.

As mentioned above, the mapping between the user-selected region(s) and the stimulation candidate region(s) can be based on a SWI image and/or a probabilistic tractography model.

Further, according to an example embodiment, the mapping can be based on response information indicating prior responses by the patient to previous neuromodulations. For example, according to an example embodiment of the present invention, the system includes sensors for sensing patient responses to stimulation, and the system is configured to create a patient-specific neural site interaction model of expected activation and inhibition based on the sensed information. For example, the system uses a stimulation paradigm (which can include various parameters, waveforms, and/or temporal patterns) to perform a stimulation and evaluates a response to the stimulation using some characteristic, e.g., the power in a certain part of the spectra of the recorded signal. (The paradigm used can be manually selected or can be preprogrammed into the system based on an understanding of best practices.) The evaluated paradigm can include multi-site stimulation. Thus, example embodiments provide a system that generates a patient-specific response model based on biopotential responses to stimulation, tractography, fMRI, PET, source-localization (i.e., using recordings, estimating sources that caused sensed signals), and/or other data.

In an example embodiment, the system includes an automated algorithm for stimulating a defined set of sites, one at a time (and, according to an example embodiment, repeatedly with different parameters), e.g., using current steering (see, e.g., U.S. Prov. Pat. App. Ser. No. 61/753,232, filed January 16, 2013 and U.S. Pat. App. Ser. No. 14/011,870, filed August 28, 2013, which published as U.S. Pat. App. Pub. No. 2014/0066999), and sensing the evoked responses to the stimulation at one or more of the defined set of sites, to create an interaction model specific to that patient. The automated algorithm, causing the processor to trigger the pulses for the stimulation, can be executed under the watchful eye of a clinician, and can include causing brief stimulations where response latencies permit.

The sensed responses, and therefore the sensing, can be at sites other than those being stimulated. In other words, stimulation at location A can cause a response at location B. According to an example embodiment, the algorithm includes automated evaluation of simultaneous multi-site stimulation and sensing at one or more sites, to expand the interaction model. The number of multi-site stimulation combinations may be large, and automatic testing of all of the possible combinations can be inefficient. Therefore, according to an example embodiment, the system gives the user the capability to choose specific types of stimulation (e.g., only anodic monopolar stimulation, only cathodic monopolar stimulation, with particular pulse widths, certain specified frequencies of pulses, etc.) to include for the algorithmic automated evaluation, and is also given the capability to manually prescribe specific stimulation configurations to test and add to the neural system interaction model.

According to an example embodiment, the system provides an "inverse" capability, whereby the system obtains user-input of an identification of a desired system-level response to stimulation (e.g., excite one or more neural areas, and/or inhibit one or more neural areas), and the system executes an algorithm to do one or more of the following: (1) evaluate stimulation paradigms (via evoked responses sensed by the system), search the stimulation parameter space (stimulation sites, parameters such as PW, rate, amplitude, configuration, and spatio-temporal stimulation paradigms between sites) for a stimulation paradigm that achieves the user-described stimulation objective, and output the determined paradigm with stimulation parameters to the user, i.e., the system iterativly tries new paradigms and parameters until the system achieves or gets close to the input goal, which can be determined based on sensor and/or user input data; (2) use the interaction model (radiographicallyderived and/or evoked potential-derived, as described above) and determine and provide to the user a stimulation paradigm (could be single-site or multi-site with independent stimulation parameters for each site, and the spatio-temporal relationship of stimulation, i.e., when to stimulate where, may be part of the paradigm), i.e., use the interaction model to estimate which stimulation sites and settings would result in the desired goal; and (3) provide information, such as a visualization of the stimulation to response mapping, the interaction matrix, etc., to the user for evaluating the paradigms and searching for stimulation parameters. Note that for parameter spaces that are particularly vast, and/or for systems that are nonlinear, genetic optimization algorithms and/or support vector machine-based algorithms are good candidates for use for selecting a stimulation paradigm and associated parameters.

Thus, according to an example, the processor generates the model by performing a plurality of neuromodulations at a respective plurality of sets of neuromodulation settings; and recording, for each of at least a subset of the neurmodulations, at least one neuromodulation effect occurring at a respective one or more anatomical regions, the one or more anatomical region being responsively mapped by the processor to a respective one or more stimulation regions of tissue estimated by the processor to have been activated by the respective neuromodulation. However, in an example, the mapping performed by the processor between a target anatomical stimulation candidate region and user-selected neuromodulation effect region is performed by extrapolating new stimulation parameters from modeled stimulation parameters that are mapped in the model to neuromodulation effect regions, which stimulation parameters are estimated, by the processor and based on the model, to produce a selected effect at the selected neuromodulation effect region, and the selected effect at the selected neuromodulation effect region need not be mapped in the model itself.

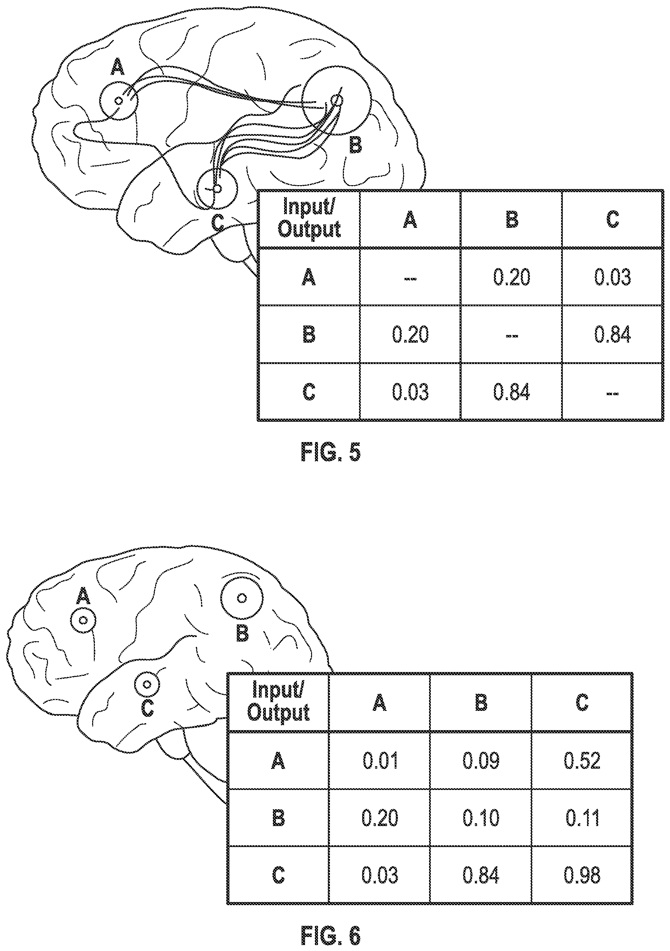

For example, Fig. 6 shows an example patient-specific interaction model, where the rows represent the stimulation sites and the columns represent the resulting neuromodulation effect at various sites. For example, row A shows that a stimulation at a particular parameter set at site A produces a change in a particular neuromodulation effect (e.g., local field potential (LFP), power at a particular frequency band, and/or neuro-activation spike) at each of sites A, B, and C, where the change is minimal at sites A and B (0.01 and 0.09, respectively) but larger at site C (0.52). Rows B and C similarly show changes occurring at sites A-C in response to stimulations at sites B and C, respectively.

In an example embodiment, the system initially applies assumptions of linearity of those relationships with respect to stimulation parameters (an increase in stimulation amplitude, for example, is assumed to cause a linear increase in the indicated changes) and/or with respect to combinations (stimulation at sites A and B assumed to produce an additive effect of the stimulations at sites A and B individually). After obtaining candidate stimulation sites and parameters based on the model with the linearity assumptions, the system applied a suitably appropriate optimization algorithm, e.g., a conventionally known gradient descent optimization routine or variation thereof, to further refine the suggested site(s) and parameter(s) to be used for achieving the target neuromodulation.

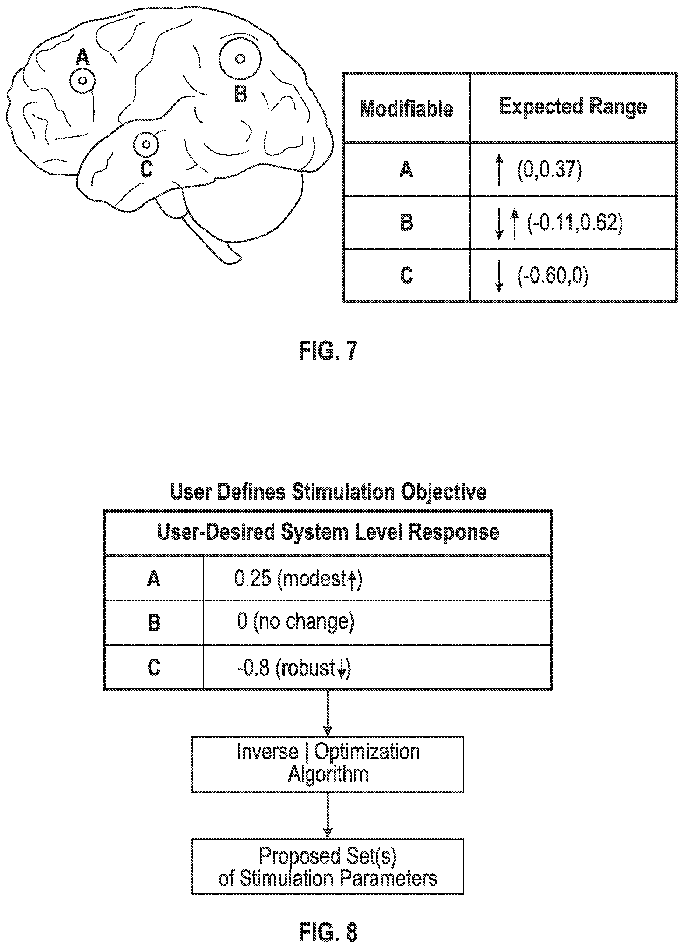

Fig. 8 shows a data flow of a method for outputting suggested stimulation sites and/or parameters for input target neuromodulation sites. Fig. 8 shows that a user defines a stimulation objective, e.g., to increase and/or reduce neural activity in one or more respective regions. In the example shown in Fig. 8, the user inputs a target of a modest increase in a particular defined characteristic (there can be any number of characteristics, such as LFP or spikes) at site A, no change at site B, and a robust inhibitory change at site C. The system then responsively performs an inverse optimization algorithm, as described, to determine suggested stimulation sites and/or parameters to achieve the input target neuromodulation effect at the indicated target sites, and then outputs the determined sites and/or parameters as a suggestion to the user.

It is noted that the patient-specific interaction model (alone or in combination with the optimization algorithm) can indicate multiple ways by which to produce the targeted neuromodulation, but it can occur that one way involves stimulation at more sites than another way, in which case, according to an example embodiment, the system takes this into consideration when suggesting a stimulation paradigm, stimulation at fewer sites being considered more optimal all else being equal.

According to an example embodiment, the development of interaction models and the determination of the solution to the "inverse" problem are automated so that the physician/clinician/user would only define the system-level stimulation goal, and the system would automatically determine and output to the user a desired stimulation paradigm (which may be very complex).

**In** an example embodiment of the present invention, the system outputs a user interface display that shows the user which parts of the neural system likely are able to be affected (an ability-to-impact model), for example, based on the interaction models. Such output can give the user a sense of constraints when defining the stimulation goal (for a given implanted system). When the interaction model is non-invasively derived, e.g., via tractography, functional imaging, etc., this output can be helpful for surgical planning, where the ability-to-impact model is specific to candidate electrode sites. For example, the system can output the ability-to-impact model for each of a plurality of leadwire sites input by the user, and provide the ability-to-impact model for each of the input leadwire sites. The user can then select whichever site provides the best possible coverage.

According to an example embodiment, the user can define a stimulation goal and set the system to run in an open-loop mode. The user can also alternatively set a stimulation goal to dynamically change responsive to a sensed system state. For example, the user can define one or more sensed system states, and one or more respective neural system stimulation goals (with corresponding stimulation paradigms), and define which stimulation paradigm is triggered for a given sensed system state.

The system provides tools for the user to define a sensed system state. The state can be defined by feedback parameters including, for example, sensor data (e.g., of external sensors wirelessly connected to the system) and/or user feedback (e.g., patient feedback input via a remote control). For example, according to an example embodiment, the power or changes in power in parts or all of the measured frequency spectrum at specific sites (e.g., the beta power in area A is high, and the gamma power in area D is also high) is one way of defining a sensed system state. Additionally or alternatively, the user can set the system to perform time-domain analyses for obtaining a system state on which basis to dynamically change the stimulation goal. Such analyses can include, for example, wavelet analysis, statistical measures, properties of single-unit firing patterns, i.e., firing patterns of action potentials of a single neuron, (e.g., inter-spike-interval relationships, i.e., the time between action potential and the patterns of such intervals, and/or other relationships concerning a neuron measureable with small electrodes).

A non-exhaustive list of example sensors used for system operation (based on the raw or processed data from the sensors) includes bio-potential sensor, chemical (e.g., neurotransmitter) sensors, temperature sensors, and physical sensors such as accelerometers, goniometers, etc. that may or may not be implanted. For example, in an example embodiment one or more external sensors are connected to the system wirelessly. In some embodiments, data sensed by these sensors contributes to (or comprises) the system interaction model on which basis the user can define stimulation goals (as described above). In some embodiments, the data sensed by these sensors alternatively or additionally provides a sensed system state, for example, for the closed-loop control.

Thus, for example, according to an example, a method includes a system controlling electrodes of an implanted leadwire to perform a neuromodulation therapy according to a set of stimulation parameters, obtaining sensor readings from the electrodes indicating a patient state, and, responsive to the state indicated by the sensor readings and based on the user-input state information, modifying the stimulation parameters.

Further, according to an example, the system receives over time a plurality of user input of clinical states and correlates the user input clinical states to biopotential signatures sensed by electrodes at times to which the clinical states respectively correspond, where the sensor readings responsive to which the stimulation parameters are modified indicate respective biopotential signatures, and the modification is based on the correlation.

In an example embodiment of the invention, data from non-implanted sensors (e.g., a blood pressure sensor) are time-correlated to data from implanted sensors using an algorithm (e.g., a support vector machine or artificial neural network), such that the user can define the sensed system state via external sensors, and a surrogate sensed system state based on sensing done by the implantable sensors can be defined (e.g., automatically) and used in the absence of external sensing tools. For example, the signal from external sensors is correlated to a measurement by the implanted system, and if there is good correlation, then the signal measured by the implanted system can be used instead. Thereafter, the sensed measurement from the electrodes of the implanted leadwire can be used for the closed-loop control.

Some example sensor readings which can be correlated by the system with states on which basis to modify the settings include mean firing rate, power readings at a specified frequency band, and local field potentials.

In an example embodiment, the system is configured to provide an interface via which a patient can define a state of being (e.g., a pain score, or a depression score, or a stress/anxiety score) at several points in time, and the system searches via algorithms for a sensed system state via the implantable sensors that correlates with the patient scores. That is, the system looks for signatures in the sensed data (e.g., biopotentials, such as voltages generated by biological systems, e.g., LFPs and action potentials) that correlate with the patient input about the patient's state. The sensor data and/or patient input can be provided in one or more sessions where an algorithm collects "training" data by which the biopotential signature-to-state relationships are determined. In an example embodiment, the algorithm is a dynamic learning algorithm that continuously updates as new "training" data is obtained via patient feedback (these algorithms can be particularly useful given the possibility and perhaps likelihood of plastic change in the neural system over time). Stimulation can then be set to be responsive to the sensed system bipotential state. Thus, based on the training data, the system associates biopotentials with particular patient states, and thereafter, the system can change which stimulation paradigms and/or stimulation settings in response to the biopotential state. Automated algorithms for defining the sensed system state are particularly useful because they, as noted above, make a system of such vast complexity usable in clinical practice.

For example, the system is configured to obtain from a patient and overtime, for each of a plurality of stimulation sessions, feedback, e.g., OK, Mild, Medium, Severe, etc., regarding particular symptoms, e.g., depression, stress, pain, etc., and, based on such feedback, update the preferred stimulation paradigm and parameters. Moreover, the feedback can be associated with biopotentials, which biotential signatures can serve as system states with which different stimulation paradigms are associated, so that the system changes which paradigm and settings are used at different sensed biopotentials. In an example embodiment, the system is configured to automatically stimulate using the updated preferred stimulation paradigm and parameters as long as the preferred program is within user-defined parameter constraints.

In an example embodiment, the system uses multiple independent current control (i.e., multiple current sources), thereby providing for a more stable stimulation, and a more finetuned stimulation (enables "virtual contacts" so that many stimulation sites can be defined with a fewer numbers of electrodes). The "virtual contacts" also enable higher resolution mapping of the neural system by centering stimulation at positions of the leadwire at which an electrode does not exist.

As described above, according to an example, the present system uses a patient-specific interaction biopotential analytics-based model or tractographical model to output a suggested stimulation paradigm for an input neuromodulation target. This significantly simplifies the setting by the clinician of stimulation parameters so that, according to example embodiments, the system can include one or more leadwires with numerous electrodes, e.g., 32 or more electrodes, even as many as into the hundreds of electrodes, since it is not required for the clinician to select which of the electrodes to use or the parameters to be used without any guidance on the stimulation regions on which to focus. Instead, the system can generate the patient-specific interaction model for a system including many electrodes. The user can input the target neuromodulation sites and effects, and, based on the model, the system outputs suggested combination of electrodes (at respective stimulation sites) and the respective simulation parameters for effecting the target neuromodulation response. (It is noted that the suggested stimulation paradigm can include a sequence of stimulations using different combinations of electrodes at different settings at different points throughout the sequence.)

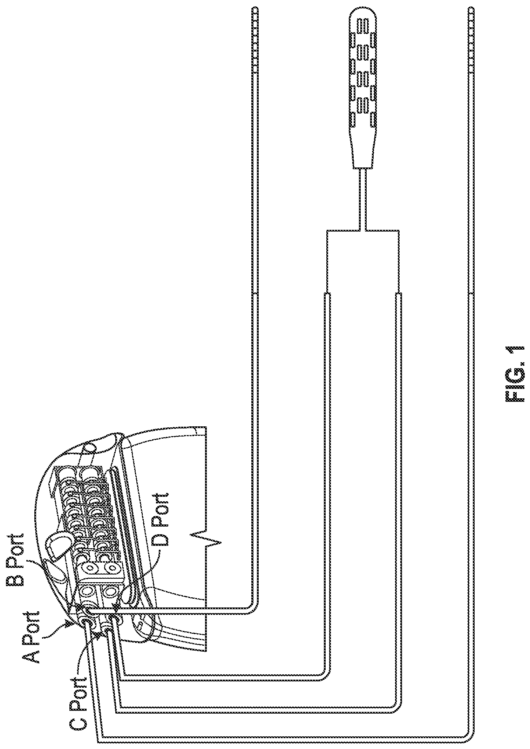

Accordingly, Fig. 1 shows components of a system according to an example embodiment of the present invention, including an IPG with four ports A-D, each of which is shown with a wired connection to a respective set of 8 contacts. The respective eight contacts to which ports A and B are connected are on respective percutaneous cylindrical leads that are implanted, e.g., into the brain. The two sets of eight contacts to which ports C and D are connected are shown to be on a single paddle lead, i.e., the paddle lead includes 16 electrodes to which the IPG is connected via ports C and D. The paddle lead can be, according to an example embodiment, adapted for implantation on top of the brain.

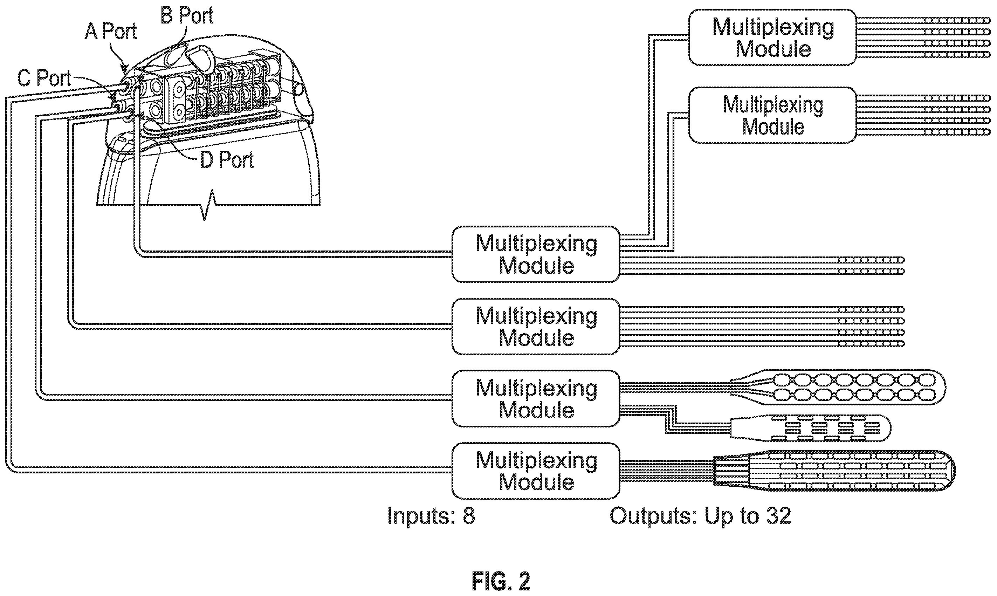

Fig. 2 shows another example embodiment including 22 leadwires with a total of 176 electrodes, where the IPG is adapted for controlling 32 of the electrodes at any one time. The illustrated IPG includes four ports A-D each providing 8 inputs to a respective multiplexing module. Each of the multiplexing modules selects which of up to 8 electrodes, of a plurality of electrodes to which the respective multiplexing module is connected, to control based on the input from the IPG. This embodiment assumes that the system would select between various combinations of the 176 electrodes for achieving a target, where any output paradigm would include stimulation using, at any one time, only up to 32 electrodes. The paradigm can, however, include a sequence where different combinations of up to 32 electrodes from the 176 electrodes are used at different times, the paradigm potentially making use of all 176 electrodes. Additionally, these numbers are provided by way of example. Other embodiments may include a different number of total electrodes and may allow for a different number of the electrodes to be used simultaneously, e.g., more than 32.

Thus, according to an example embodiment of the present invention, a stimulation system includes an implanted pulse generator (IPG) including a plurality of ports; a plurality of leadwires, each of the leadwires including at least one respective electrode; and at least one multiplexer between a respective one of the ports and a respective subset of more than one of the plurality of leadwires, which multiplexer is configured to select between the subset of the more than one of the plurality of leadwires for application to the electrodes thereof, of a signal from the respective port, the signal causing the electrodes thereof to produce electrical pulses for neuromodulation of tissue in which the subset of leadwires is implanted.

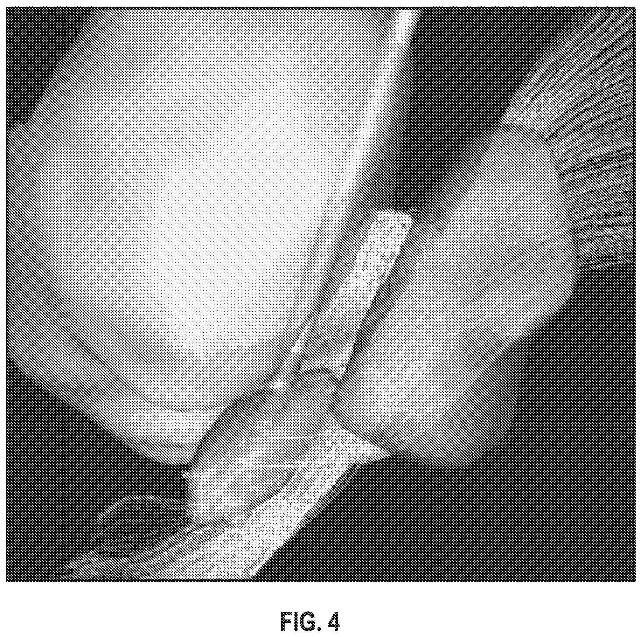

Fig. 4 shows information captured in a tractography map, which can be used to understand relationships between different parts of brain by analyzing fiber paths from the image, as discussed above. Fig. 5 shows an example probabilistic tractographically based stimulation to neuromodulation mapping. For example, in an example embodiment, based on a tractography map, such as that shown in Fig. 4, the system determines that a stimulation at one region is expected to cause a neuromodulation response at another region (and vice versa) because of the extension of a neural element between those two regions. As described above, a probabilistic tractographic interaction model can be used instead of a biopotential response based model.

In an example embodiment, the probabilistic tractographical neuro modulation response mapping is based on a tractography map, such as one shown in Fig. 4, alone. In an alternative example embodiment, the probabilistic tractographical neuro modulation response mapping is additionally based on neural modeling including a model of a produced electric field in a patient anatomy at particular stimulation parameters and where such an electric field reaches a threshold for causing a neural activation. Combining such a neural model with a tractography map provides further refined data. Specifically, according to an example embodiment, the system uses the neural model to determine which neural elements would be activated at a particular stimulation paradigm, and then uses the tractography map to determine the neuromodulation sites that would be affected by those activated neural elements. For example, if the system determines from the neural model that site A would be activated, and determines that a particular neural element located at site A extends to site B, the system would estimate that site B would also be activated.

The above description is intended to be illustrative, and not restrictive. Those skilled in the art can appreciate from the foregoing description that the present invention may be implemented in a variety of forms, and that the various embodiments may be implemented alone or in combination. Therefore, while the embodiments of the present invention have been described in connection with particular examples thereof, the true scope of the claimed invention is defined by the appended set of claims.

## Claims

1. A computer-implemented method comprising:
generating or obtaining a mapping between neuromodulation effect regions (A-C) of a patient and target anatomical stimulation candidate regions;
receiving, by a computer processor, user input selecting one of the neuromodulation effect regions (A-C) of the patient;
using, by the computer processor, the mapping to map at least one target anatomical stimulation candidate region to the selected one of the neuromodulation effect regions; and
responsive to the user input, determining, by the computer processor, electrode neuromodulation settings estimated by the computer processor to, when applied to an implanted electrode leadwire, produce a volume of tissue activation that at least partially encompasses the at least one target anatomical stimulation candidate region which is mapped by the computer processor to, and is at a distance from, the selected one of the neuromodulation effect regions (A-C);
wherein the mapping:
is based on a probabilistic tractography model which is based on a susceptibility weighted imaging, SWI, image, wherein the computer processor performs an estimation of stimulation effect based on the probabilistic tractography model; and/or
includes a patient-specific mapping that is specific to a patient and that is based on response information indicating prior responses by the patient to previous neuromodulations, wherein the response information includes sensor information sensed by at least one sensor configured for sensing patient responses to stimulation.

2. The method of claim 1, wherein the at least one target anatomical stimulation candidate region includes a plurality of distinct regions.

3. The method of any of the preceding claims, wherein the user input specifies the neuromodulation effect region (A-C) as a region at which neural activity is to be reduced.

4. The method of any of the preceding claims, wherein the selected neuromodulation effect region (A-C) is one of a plurality of neuromodulation effect regions (A-C) selected by the user input, and the determining includes determining one or more sets of neuromodulation settings to be distributed to one or more electrodes of the leadwire to produce a selected therapeutic effect at the plurality of neuromodulation effect regions (A-C).

5. The method of any of the preceding claims wherein the user input selects a neuromodulation effect region (A-C) in a brain of the patient.

6. A processor configured to perform the method of any of the preceding claims.

7. A system, comprising:
an implanted pulse generator, IPG;
a processor configured to perform the method of any of claims 1 to 5 and to provide the electrode neuromodulation settings to the IPG; and
at least one sensor configured to sense patient responses to stimulation and to provide sensor information to the processor.

8. The system of claim 7, wherein the processor is configured to modify the electrode neuromodulation settings using the sensor information.

9. The system of claim 7, wherein at least one of the at least one sensor is configured to measure at least one of a mean firing rate of neural elements, power at a specified frequency band, or a local field potential.

## Patentansprüche

1. Computerimplementiertes Verfahren, umfassend:
Erzeugen oder Erhalten einer Zuordnung zwischen Neuromodulationswirkungsbereichen (A-C) eines Patienten und Ziel-anatomischen-Stimulationskandidatenbereichen;
Empfangen, durch einen Computerprozessor, einer Benutzereingabe, die einen der Neuromodulationswirkungsbereiche (A-C) des Patienten auswählt;
Verwenden, durch den Computerprozessor, der Zuordnung, um zumindest einen Ziel-anatomischen-Stimulationskandidatenbereich dem ausgewählten der Neuromodulationswirkungsbereiche zuzuordnen; und
als Reaktion auf die Benutzereingabe, Bestimmen, durch den Computerprozessor, von Elektroden-Neuromodulationseinstellungen, die vom Computerprozessor geschätzt werden, um, wenn sie an eine implantierte Elektrodenanschlussleitung angelegt werden, ein Volumen an Gewebeaktivierung zu erzeugen, das zumindest teilweise den zumindest einen Ziel-anatomischen-Stimulationskandidatenbereich umfasst, der vom Computerprozessor dem ausgewählten der Neuromodulationswirkungsbereiche (A-C) zugeordnet wird und sich in einem Abstand zu diesem befindet;
wobei die Zuordnung:
auf einem probabilistischen Traktografiemodell basiert, das auf einem suszeptibilitätsgewichteten Bildgebungs-, SWI, Bild basiert, wobei der Computerprozessor eine Schätzung von Stimulationswirkung basierend auf dem probabilistischen Traktografiemodell durchführt; und/oder
eine patientenspezifische Zuordnung aufweist, die für einen Patienten spezifisch ist und auf Reaktionsinformationen basiert, die frühere Reaktionen des Patienten auf vorherige Neuromodulationen anzeigen, wobei die Reaktionsinformationen Sensorinformationen aufweisen, die von mindestens einem Sensor erfasst werden, der zum Erfassen von Patientenreaktionen auf Stimulation konfiguriert ist.

2. Verfahren nach Anspruch 1, wobei der zumindest eine Ziel-anatomische-Stimulationskandidatenbereich eine Mehrzahl von unterschiedlichen Bereichen aufweist.

3. Verfahren nach irgendeinem der vorhergehenden Ansprüche, wobei die Benutzereingabe den Neuromodulationswirkungsbereich (A-C) als einen Bereich spezifiziert, in dem neuronale Aktivität reduziert werden soll.

4. Verfahren nach irgendeinem der vorhergehenden Ansprüche, wobei der ausgewählte Neuromodulationswirkungsbereich (A-C) einer von einer Mehrzahl von Neuromodulationswirkungsbereichen (A-C) ist, die durch die Benutzereingabe ausgewählt werden, und das Bestimmen ein Bestimmen von einem oder mehreren Sätzen von Neuromodulationseinstellungen umfasst, die auf eine oder mehrere Elektroden der Anschlussleitung verteilt werden sollen, um eine ausgewählte therapeutische Wirkung an der Mehrzahl von Neuromodulationswirkungsbereichen (A-C) zu erzeugen.

5. Verfahren nach irgendeinem der vorhergehenden Ansprüche, wobei die Benutzereingabe einen Neuromodulationswirkungsbereich (A-C) im Gehirn des Patienten auswählt.

6. Prozessor, der konfiguriert ist, das Verfahren nach irgendeinem der vorhergehenden Ansprüche auszuführen.

7. System, aufweisend:
einen implantierten Impulsgenerator, IPG;
einen Prozessor, der konfiguriert ist, das Verfahren nach irgendeinem der Ansprüche 1 bis 5 auszuführen und die Elektroden-Neuromodulationseinstellungen an den IPG zu liefern; und
zumindest einen Sensor, der konfiguriert ist, Patientenreaktionen auf Stimulation zu erfassen und Sensorinformationen an den Prozessor zu liefern.

8. System nach Anspruch 7, wobei der Prozessor konfiguriert ist, die Elektroden-Neuromodulationseinstellungen unter Verwendung der Sensorinformationen zu modifizieren.

9. System nach Anspruch 7, wobei zumindest einer des zumindest einen Sensors konfiguriert ist, zumindest eines aus einer mittleren Feuerrate neuronaler Elemente, einer Leistung in einem bestimmten Frequenzband, oder eines lokalen Feldpotentials zu messen.

## Revendications

1. Procédé mis en œuvre par ordinateur comprenant les étapes suivantes :
générer ou obtenir un mappage entre des régions d'effet de neuromodulation (A-C) d'un patient et des régions de stimulations anatomiques candidates cibles ;
recevoir par un processeur informatique une entrée utilisateur sélectionnant une des régions d'effet de neuromodulation (A-C) du patient ;
utiliser par le processeur informatique le mappage pour mapper au moins une région de stimulation anatomique candidate cible à cette région sélectionnée des régions d'effet de neuromodulation ; et
en réponse à l'entrée utilisateur, déterminer par le processeur informatique des réglages de neuromodulation par électrode estimés par le processeur informatique pour, lorsqu'ils sont appliqués à un fil conducteur d'électrode implanté, produire un volume d'une activation tissulaire qui englobe au moins partiellement l'au moins une région de stimulation anatomique candidate cible mappée par le processeur informatique à cette région sélectionnée des régions d'effet de neuromodulation (A-C) et se trouvant à une distance de celle-ci ;
dans lequel le mappage :
est basé sur un modèle de tractographie probabiliste qui est basé sur une image d'imagerie pondérée par la susceptibilité, SWI, dans lequel le processeur informatique effectue une estimation d'un effet de stimulation sur la base du modèle de tractographie probabiliste ; et/ou
comporte un mappage spécifique au patient qui est spécifique à un patient et qui est basé sur des informations de réponse indiquant des réponses antérieures par le patient à des neuromodulations précédentes, dans lequel les informations de réponse comportent des informations de capteur détectées par au moins un capteur configuré pour détecter des réponses de patient à une stimulation.

2. Procédé selon la revendication 1, dans lequel la au moins une région de stimulation anatomique candidate cible comporte une pluralité de régions distinctes.

3. Procédé selon l'une des revendications précédentes, dans lequel l'entrée utilisateur spécifie la région d'effet de neuromodulation (A-C) comme une région dans laquelle l'activité neuronale doit être réduite.

4. Procédé selon l'une des revendications précédentes, dans lequel la région d'effet de neuromodulation (A-C) sélectionnée est l'une d'une pluralité de régions d'effet de neuromodulation (A-C) sélectionnées par l'entrée utilisateur, et la détermination comporte la détermination d'un ou plusieurs ensembles de réglages de neuromodulation à distribuer à une ou plusieurs électrodes du fil conducteur pour produire un effet thérapeutique sélectionné au niveau de la pluralité de régions d'effet de neuromodulation (A-C).

5. Procédé selon l'une des revendications précédentes, dans lequel l'entrée utilisateur sélectionne une région d'effet de neuromodulation (A-C) dans le cerveau du patient.

6. Processeur configuré pour mettre en œuvre le procédé selon l'une des revendications précédentes.

7. Système, comprenant :
un générateur d'impulsions implanté, IPG ;
un processeur configuré pour mettre en œuvre le procédé selon l'une des revendications 1 à 5 et pour fournir des réglages de neuromodulation par électrode à l'IPG ; et
au moins un capteur configuré pour détecter des réponses de patient à une stimulation et pour fournir des informations de capteur au processeur.

8. Système selon la revendication 7, dans lequel le processeur est configuré pour modifier les réglages de neuromodulation par électrode en utilisant les informations de capteur.

9. Système selon la revendication 7, dans lequel au moins un de l'au moins un capteur est configuré pour mesurer au moins un parmi un taux de décharge moyen d'éléments neuronaux, une puissance dans une bande de fréquences spécifiée, ou un potentiel de champ local.
